# EUROPEAN PATENT APPLICATION

(11) **EP 4 704 110 A1**
(43) Date of publication of application: **04.03.2026**
(21) Application number: 25197113.1
(22) Date of filing: 20.08.2025
(51) Int. Cl.: G16H 40/63, G16H 40/67, G16H 20/17

(54) **RESTORATION OF THERAPY STATES**

(30) Priority: 29.08.2024 US 202463688656 P; 28.07.2025 US 202519283064
(71) Applicant: Medtronic MiniMed, Inc., Northridge, CA 91325 (US)
(72) Inventor: BERIAN, Jesus, Northridge, 91325 (US); CASAREZ RUIZ, Oscar B., Northridge, 91325 (US); SERRANO, Eduardo Javier, Northridge, 91325 (US); ROSALES, Miguel F., Northridge, 91325 (US)
(74) Representative: Zimmermann & Partner Patentanwälte mbB

(57) **Abstract**

Techniques for restoration of therapy states are provided. The techniques may involve receiving, at a controller device, an indication that a therapy state used in conjunction with a first medical device is to be transferred to a second medical device. The techniques may further involve receiving, at the controller device from a cloud device, information indicative of the therapy state used in conjunction with the first medical device. The techniques may further involve transmitting, from the controller device, the information indicative of the therapy state used in conjunction with the first medical device to the second medical device.

## Description

### CROSS-REFERENCE TO RELATED APPLICATION

This application claims the benefit of and priority to U.S. Provisional Application No. 63/688,656, filed August 29, 2024, entitled "RESTORATION OF THERAPY STATES," which is assigned to the assignee hereof and is hereby incorporated by reference in its entirety for all purposes.

### TECHNICAL FIELD

The present disclosure relates generally to restoration of therapy states.

### BACKGROUND

Patients may utilize medical devices to deliver medical therapy. For example, a patient may treat diabetes using an insulin pump configured to deliver insulin to the patient. In some cases, a patient may switch from a first medical device to a replacement medical device. However, it may be difficult and time-consuming for the patient to switch medical devices due to, e.g., the time required to configure and set up the replacement medical device.

### SUMMARY

Techniques for restoration of therapy states are provided.

In some embodiments, the techniques may involve receiving, at a controller device, an indication that a therapy state used in conjunction with a first medical device is to be transferred to a second medical device. The techniques may further involve receiving, at the controller device from a cloud device, information indicative of the therapy state used in conjunction with the first medical device. The techniques may further involve transmitting, from the controller device, the information indicative of the therapy state used in conjunction with the first medical device to the second medical device.

According to some embodiments, techniques may involve receiving, at a medical device, therapy state information associated with therapy provided by a previously used medical device, wherein the therapy state information is associated with a first time point at which the therapy was provided by the previously used medical device. The techniques may further involve updating the therapy state information by the medical device. The techniques may further involve providing therapy, by the medical device, in accordance with the updated therapy state information.

Further disclosed herein are techniques for restoration of therapy states. The techniques may involve receiving, at a controller device, an indication that a therapy state used in conjunction with a first medical device is to be transferred to a second medical device. The techniques may further involve receiving, at the controller device from a cloud device, information indicative of the therapy state used in conjunction with the first medical device. The techniques may further involve transmitting, from the controller device, the information indicative of the therapy state used in conjunction with the first medical device to the second medical device.

### BRIEF DESCRIPTION OF THE DRAWINGS

The above and other aspects and features of the disclosure will become more apparent in view of the following detailed description when taken in conjunction with the accompanying drawings wherein like reference numerals identify like elements.
FIG. 1 is a diagram of an example of a therapy delivery system, in accordance with aspects of the present disclosure.
FIGS. 2A-2C illustrate example information flow diagrams for transfer of therapy state information in accordance with aspects of the present disclosure.
FIG. 3 is a flowchart of an example process for transmitting therapy state information by a controller device to a medical device in accordance with aspects of the present disclosure.
FIG. 4 is a flowchart of an example process for receiving therapy state information by a medical device and utilizing the received therapy state information to provide therapy in accordance with aspects of the present disclosure.
FIG. 5 is a diagram of an example of an insulin delivery device, in accordance with aspects of the present disclosure.
FIG. 6 is a block diagram of an example of a computer system, which can be utilized in embodiments as described herein.

### DETAILED DESCRIPTION

Therapeutic substances (e.g., insulin) may be delivered to a diabetic patient to manage, for example, type I or type II diabetes. Delivering appropriate amounts of a therapeutic substance at appropriate times can help maintain glucose levels within a target range (e.g., a euglycemic range) in the body to prevent hyperglycemia or hypoglycemia conditions.

Patients may utilize medical devices to deliver medical therapy. For example, a patient may treat diabetes using an insulin pump configured to deliver insulin to the patient. In some cases, a patient may switch from a first medical device to a replacement medical device. However, it may be difficult and time-consuming for the patient to switch medical devices due to, e.g., the time required to configure and set up the replacement medical device. For example, when a patient switches from a first insulin pump to a second insulin pump, using conventional techniques, the patient may have to reconfigure the second insulin pump to connect with a particular paired glucose sensor, to initialize particular insulin delivery algorithms, etc. This set-up process may be time-consuming, which may cause delays before a replacement medical device can be utilized to provide therapy to the patient. The delay may cause the patient to miss out on delivered medical therapy. Moreover, because set-up of the replacement medical device is conventionally performed manually, mistakes in configuring the replacement medical device may cause unintended problems.

Disclosed herein are techniques for storing therapy state information corresponding to therapy provided by a first medical device. The therapy state information may be stored as a therapy state data asset. The therapy state information may be stored by a paired controller device and/or a remote cloud device. The therapy state information may be transmitted to a replacement medical device (e.g., from a controller device and/or from the remote cloud device), e.g., at a time when the replacement medical device is to replace the first medical device in providing medical therapy to the patient. The replacement medical device may receive the therapy state information and may begin to provide therapy to the patient based at least in part on the received therapy state information. The techniques disclosed herein may allow a replacement medical device to be put into service without time-consuming configuration of the replacement medical device. Moreover, the techniques disclosed herein may abstract parameters associated with the therapy state into a format that may be stored by a controller device and/or a remote cloud device and provided to replacement medical device regardless of the type or model of the replacement medical device. This may allow for compatibility across different make/models of medical devices. Note that a controller device and/or a remote cloud device may serve to backup a therapy state used by a medical device and then provide information indicative of the most recent therapy state to a replacement medical device.

It should be noted that "therapy state information" and "information indicative of therapy state" are generally used interchangeably herein. Therapy state information may be represented as and/or stored as "a therapy state data asset." A therapy state data asset may have a defined structure that is utilized by a controller device, a remote cloud device, and/or compatible medical devices. A therapy state data asset may include multiple structural objects (e.g., "structs"), where each structural object relates to a therapy parameter and includes one or more fields having values representing the therapy state at the time the therapy state data asset was generated. Structural objects are described below in more detail. In some embodiments, a non-compatible device may be considered a medical device that is not configured to utilize a therapy state data asset. Such non-compatible devices may transmit therapy state information in other format to a controller device and/or a remote cloud device, which may re-format the received therapy state information to a therapy state data asset for storage and/or future usage.

It should be understood that although the techniques described here are generally described in the context of medical devices that provide medical treatment, the techniques may be utilized to transfer therapy state information from a first medical device to a second, replacement medical device for devices which provide treatment for any suitable condition. Examples include pain control devices, hearing devices, etc.

The present disclosure is described primarily with respect to insulin delivery systems. Aspects and embodiments of the present disclosure can be practiced with one or more types of insulin (e.g., fast-acting insulin, intermediate-acting insulin, and/or slow-acting insulin). For example, fast-acting insulin may be used for both basal dosages and bolus dosages.

Although the present disclosure is described primarily with respect to insulin delivery systems, the scope of the present disclosure is not limited to insulin delivery systems. Rather, the present disclosure applies to and can be implemented for other therapy systems as well. For example, some techniques of the present disclosure may be adapted for practice in relation to glucagon delivery systems.

Discussions utilizing terms such as, for example, "processing," "computing," "calculating," "determining," "establishing," "analyzing," "checking," or the like, may refer to operation(s) and/or process(es) of a computer, a computing platform, a computing system, or other electronic computing device, that manipulate and/or transform data represented as physical (e.g., electronic) quantities within the computer's registers and/or memories into other data similarly represented as physical quantities within the computer's registers and/or memories or other non-transitory information storage media that may store instructions to perform operations and/or processes by, e.g., one or more processor or processor apparatus (e.g., system on a chip) or a device associated with such processor(s).

In the context of this disclosure, a "module" may refer to a set of computer-executable instructions and/or a hardware processor configured to execute a set of computer-executable instructions. A hardware processor may be an integrated circuit device associated with a computing device, such as a server or a user device (e.g., a desktop computer, a laptop computer, a tablet computer, a mobile phone, or the like), which is programmable to perform specific tasks. In some embodiments, multiple modules may be implemented as a single module. In some embodiments, a single module may be implemented as multiple modules. In some embodiments, two or more modules may be executable by the same device (e.g., the same computing device or delivery device).

Unless explicitly stated, the methods described herein are not constrained to a particular order or sequence. Additionally, some of the described methods or elements thereof can occur or be performed simultaneously or concurrently.

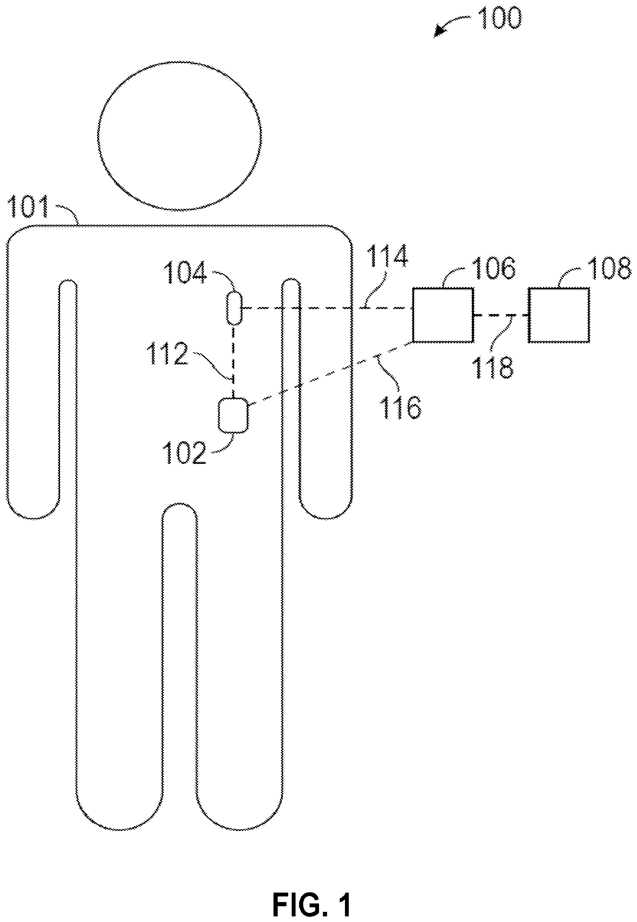

FIG. 1 depicts an example therapy delivery system 100 for a person 101. Components of therapy delivery system 100 may be used to implement one or more blocks of process 300 and/or process 400. System 100 may be an insulin delivery system. The depicted therapy delivery system 100 includes a delivery device 102, a monitoring device 104, a computing device 106, and an optional remote or cloud computing system 108. The delivery device 102, the monitoring device 104, and the computing device 106 may be embodied in various ways, including being disposed in one or more device housings. For example, in some embodiments, all of the devices 102-106 may be disposed in a single device housing. In some embodiments, each of the devices 102-106 may be disposed in a separate device housing. In some embodiments, two or more of the devices 102-106 may be disposed in the same device housing, and/or a single device 102, 104, or 106 may have two or more parts that are disposed in two or more housings. Such embodiments, and combinations thereof, are contemplated to be within the scope of the present disclosure. Note that the locations of each device shown in FIG. 1 is merely one example. For example, monitoring device 104 is depicted on a chest of the patient, however, in some implementations, monitoring device 104 may be on an arm of the patient.

FIG. 1 also depicts communications links 112-118. The communications links 112-118 may each be a wired connection and/or a wireless connection. In the case where two devices are located in the same device housing, the communication link may include, for example, wires, cables, and/or communication buses on a printed circuit board, among other things. In the case where two devices are separated from each other in different device housings, the communication links may be wired and/or wireless connections. Wired connections may include, without limitation, an Ethernet connection, a USB connection, and/or another type of physical connection. Wireless connections may include, without limitation, a cellular connection, a Wi-Fi connection, a Bluetooth^{®} connection, a mesh network connection, and/or another type of connection using a wireless communication protocol. Some embodiments of the communication links 112-118 may use direct connections, such as Bluetooth^{®} connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for the communication links 112-118.

Aspects of the insulin delivery system 100 are described below. Further aspects and details may be described in United States Patent Nos.: 4,562,751; 4,685,903; 5,080,653; 5,505,709; 5,097,122; 6,485,465; 6,554,798; 6,558,320; 6,558,351; 6,641,533; 6,659,980; 6,752,787; 6,817,990; 6,932,584; and 7,621,893. The entire contents of each of the foregoing United States Patents are hereby incorporated by reference herein.

The delivery device 102 is configured to deliver a therapeutic substance (e.g., insulin) to a person 101. The delivery device 102 may be secured to the person 101 (e.g., to the body or clothing of the person 101) or may be implanted on or in the body of the person 101. In some embodiments, the delivery device 102 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of the person 101. The cannula and/or a needle may facilitate delivery of the therapeutic substance to a tissue layer, vein, or body cavity of the person 101. During operation, the actuator, in response to a signal (e.g., a command signal), may drive the delivery mechanism, thereby causing the therapeutic substance to move from the reservoir, through the cannula, and into the body of the person 101.

The components of the delivery device 102 described above are merely provided as examples. The delivery device 102 may include other components, such as, without limitation, a power supply, a communication transceiver, computing resources, and/or user interfaces, among other things. Persons skilled in the art will recognize various implementations of the delivery device 102 and the components of such implementations. All such implementations and components are contemplated to be within the scope of the present disclosure.

With continuing reference to FIG. 1, the monitoring device 104 is configured to detect a physiological condition (e.g., a glucose concentration level) of the person 101 and may also be configured to detect other things. The monitoring device 104 may be secured to the body of the person 101 (e.g., to the skin of person 101 via an adhesive) and/or may be at least partially implanted into the body of the person 101. Depending on the particular location or configuration, the monitoring device 104 may be in contact with biological matter (e.g., interstitial fluid and/or blood) of the person 101.

The monitoring device 104 includes one or more sensors (not shown), such as, without limitation, electrochemical sensors, electrical sensors, and/or optical sensors. As persons skilled in the art will understand, an electrochemical sensor may be configured to respond to the interaction or binding of a biological marker to a substrate by generating an electrical signal based on a potential, conductance, and/or impedance of the substrate. The substrate may include a material selected to interact with a particular biomarker, such as glucose. The potential, conductance, and/or impedance may be proportional to a concentration of the particular biomarker. In the case of electrical sensors, and as persons skilled in the art will understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of the person 101. In some embodiments, the electrical biosignal may include portions of a potential change produced by the heart of the person 101 over time, e.g., recorded as an electrocardiogram, that are indicative of a glucose level of the person 101. In the case of optical sensors, as persons skilled in the art will understand, an optical sensor may be configured to respond to the interaction or binding of a biological marker to a substrate by generating an electrical signal based on change in luminance of the substrate. For example, the substrate may include a material selected to fluoresce in response to contact with a selected biomarker, such as glucose. The fluorescence may be proportional to a concentration of the selected biomarker.

In some embodiments, the monitoring device 104 may include other types of sensors that may be worn, carried, or coupled to the person 101 to measure activity of the person 101 that may influence the glucose levels or glycemic response of the person 101. As an example, the sensors may include an acceleration sensor configured to detect an acceleration of the person 101 or a portion of the person 101, such as the person's hands or feet. The acceleration (or lack thereof) may be indicative of exercise, sleep, or food/beverage consumption activity of the person 101, which may influence the glycemic response of the person 101. In some embodiments, the sensors may include heart rate and/or body temperature, which may indicate an amount of physical exertion experienced by the person 101. In some embodiments, the sensors may include a GPS receiver which detects GPS signals to determine a location of the person 101.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor shall be referred to as a "sensor signal."

The monitoring device 104 may include components and/or circuitry configured to pre-process sensor signals. Pre-processing may include, without limitation, amplification, filtering, attenuation, scaling, isolation, normalization, transformation, sampling, and/or analog-to-digital conversion, among other things. Persons skilled in the art will recognize various implementations for such pre-processing, including, without limitation, implementations using processors, controllers, ASICS, integrated circuits, hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, the monitoring device 104 may not perform pre-processing.

As used herein, the term "sensed data" shall mean and include the information represented by a sensor signal or by a pre-processed sensor signal. In some embodiments, sensed data may include glucose levels in a person 101, acceleration of a part of the person 101, heart rate of the person 101, temperature of the person 101, and/or geolocation (e.g., GPS location) of the person 101, among other things. The monitoring device 104 may communicate sensed data to the delivery device 102 via communication link 112 and/or to the computing device 106 via communication link 114. Use of sensed data by the delivery device 102 and/or by the computing device 106 will be described later herein.

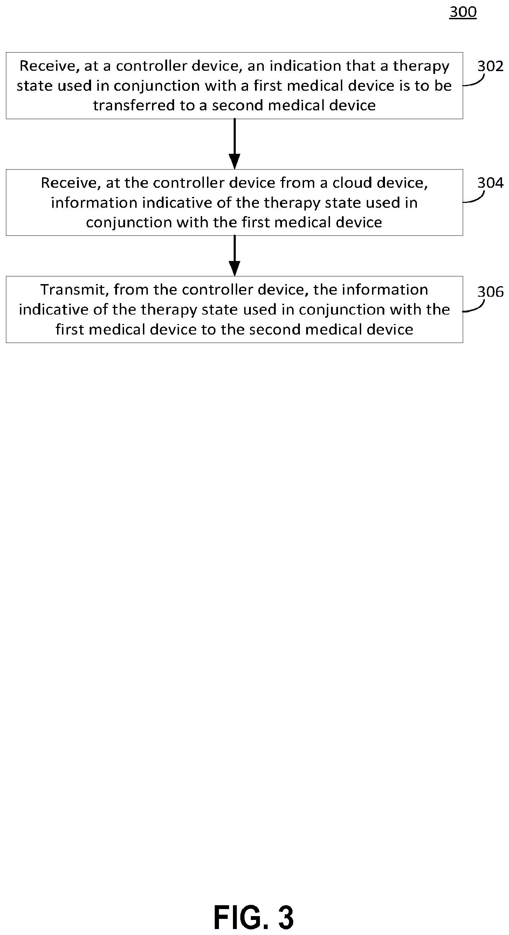

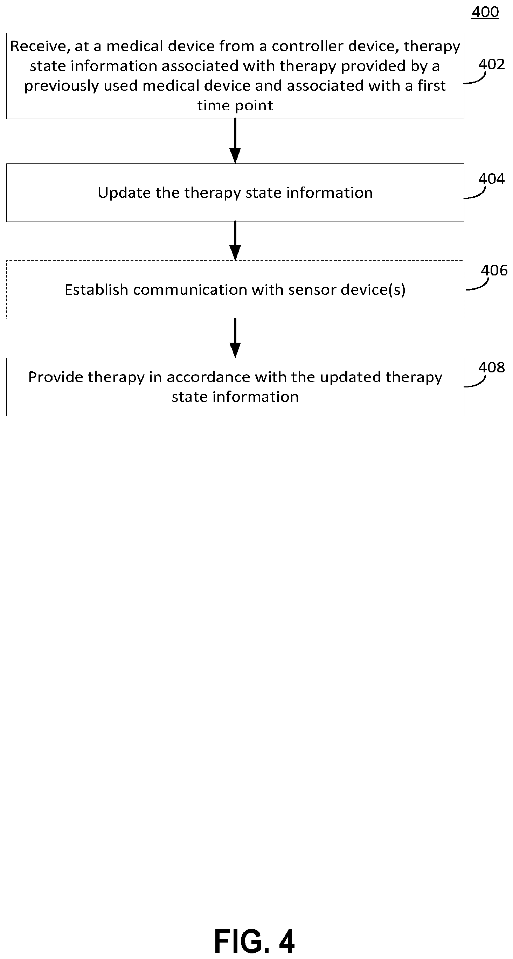

The computing device 106 provides processing capabilities and may be implemented in various ways. In some embodiments, the computing device 106 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of the delivery device 102. In some embodiments, the computing device 106 may be "processing circuitry" (defined below) that is integrated with another device, such as the delivery device 102. In some embodiments, the computing device 106 may be secured to the person 101 (e.g., to the body or clothing of person 101), may be at least partially implanted into the body of person 101, and/or may be held by the person 101. In some embodiments, computing device 106 may be configured to execute one or more blocks of process 300 and/or process 400, shown in FIGS. 3 and 4 below.

For each of the embodiments of the computing device 106, the computing device 106 may include various types of logic circuitry, including, but not limited to, microprocessors, microcontrollers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Aspects of the delivery device 102, the monitoring device 104, and the computing device 106 have been described above. One or more of the devices 102-106 may include a user interface (not shown) that presents information to the person 101 and/or receives information from the person 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where the computing device 106 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify the person 101 of sensed data (e.g., glucose level) and/or insulin delivery data (e.g., rates of historic, current, or future insulin delivery) and may present alerts to the person 101. In some embodiments, a user interface may receive inputs from the person 101, which may include, for example, a requested change in insulin delivery and/or a meal indication, among other things. The descriptions and embodiments above regarding user interfaces are merely provided as examples, and other types and other uses of user interfaces are contemplated to be within the scope of the present disclosure.

The following describes communications between the devices 102-106 and cooperation between the devices 102-106 with respect to insulin delivery. As depicted in FIG. 1, and as mentioned above, the devices 102-106 may communicate with each other via communication links 112-116. In some embodiments, the computing device 106 may control operation of the delivery device 102 and/or the monitoring device 104. For example, the computing device 106 may generate one or more signals (e.g., a command signal) that cause the delivery device 102 to deliver insulin to the person 101, e.g., as a basal dosage and/or a bolus dosage. In some embodiments, the computing device 106 may receive data associated with insulin delivery (e.g., insulin delivery data) from the delivery device 102 and/or receive sensed data (e.g., glucose levels) from the monitoring device 104 and may perform computations based on the insulin delivery data, the sensed data, and/or other data to control the delivery device 102. Insulin delivery data may include, but is not limited to, a type of insulin being delivered, historical insulin delivery rates and/or amounts, current insulin delivery rate and/or amount, and/or user input affecting insulin delivery. As persons skilled in the art will understand, in a closed-loop operating mode, computing device 106 may communicate dosage commands to the delivery device 102 based on a difference between a current glucose level in the body of the person 101 (e.g., received from the monitoring device 104) and a target glucose level (e.g., determined by the computing device 106). The dosage commands may indicate an amount of insulin to be delivered and/or a rate of insulin delivery and may regulate the current glucose level toward the target glucose level. Examples of closed-loop operations for insulin infusion systems are described in United States Patent Nos.: 6,088,608, 6,119,028, 6,589,229, 6,740,072, 6,827,702, 7,323,142, and 7,402,153, and in United States Patent Application Publication Nos.: 2014/0066887 and 2014/0066889. The entire contents of each of the foregoing patents and publications are hereby incorporated by reference herein.

With continuing reference to FIG. 1, the remote or cloud computing system 108 may be a proprietary remote/cloud computing system or a commercial cloud computing system including one or more server computing devices. The remote/cloud computing system 108 may provide additional computing resources on-demand as needed when the computing resources of a client computing device (e.g., the computing device 106) are not sufficient. The computing device 106 and the remote/cloud computing system 108 may communicate with each other through a communication link 118, which may traverse one or more communication networks (not shown). The communication networks may include, without limitation, an Ethernet network, Wi-Fi network, a cellular network, a satellite network, an intranet, an extranet, the Internet, and/or the Internet backbone, among other types of networks. Persons skilled in the art will recognize implementations for the remote/cloud computing system 108 and how to interface with such systems through various types of networks. For example, the remote/cloud computing system 108 may include an array of processing circuitry (defined above) and may execute machine-readable instructions. Such implementations, interfaces, and networks are contemplated to be within the scope of the present disclosure. In some embodiments, remote or cloud computing system 108 may be configured to execute one or more blocks of process 300 and/or process 400, as shown in FIGS. 3 and 4, respectively.

An example therapy delivery system has been described above. For convenience, the description below may primarily refer to an insulin delivery system as an example of the therapy delivery system. However, it is intended that any aspect, embodiment, or description relating to an insulin delivery system shall be applicable to a therapy delivery system which delivers a therapy other than insulin.

As described above, using the techniques described herein, a current state of therapy provided to a patient may be represented as "therapy state information," generally used herein to refer to information indicative of the therapy provided to a patient at a point in time by a medical device. In some implementations, the therapy state information may be formatted as a data asset that may be stored and transferred across medical devices such that when a patient transitions from using a first medical device (e.g., a first insulin pump) to a second medical device (e.g., a second insulin pump), the therapy state representing the therapy delivered by the first medical device may be provided to the second medical device. Accordingly, the second medical device can begin providing therapy to the patient based on the information encapsulated in the therapy state data asset without requiring user input, allowing for a seamless transition between devices. Therapy state information may include connectivity data, time management data, distributed notifications data, manual therapy data, sensor data (e.g., glucose sensor data), therapy algorithm data, or the like. Each of these aspects of a therapy state data asset are described below in more detail.

Connectivity data may include information usable to establish a connection between the medical device and one or more other devices, such as a controller device, a glucose sensor, etc. For example, the connectivity data may include information indicating a serial number of the device to be connected to (e.g., a serial number of a glucose sensor), and/or security information (e.g., a passcode) required to establish a connection between the medical device and the other device. For example, the connectivity data may include a passcode required to pair an insulin pump with a glucose sensor.

Time management data may include timestamp information. For example, the timestamp information may indicate timing information (e.g., a date, a time, etc.) at which therapy state information was created or was updated.

Distributed notifications data may include information regarding alerts and/or alarms. For example, the distributed notifications data may include a time an alert or alarm was provided, a manner in which the alert or alarm is to be provided (e.g., whether the alert/alarm is to provide haptic notifications, audible notifications, a visible alert or message, etc.), escalation information (e.g., one or more actions to be taken if the alert/alarm is not addressed, a time period during which the alert/alarm is to be addressed, etc.), or the like. By way of example, a first medical device may trigger a low glucose level alert or alarm at a first time point. The information associated with the alarm (e.g., timing information, type of alarm, escalation information, etc.) may be stored as therapy state information. In an instance in which therapy is transferred to being provided by a second medical device, the second medical device may identify the alert that was triggered by the first medical device based on the transferred therapy state information. The second medical device may then determine whether or not the alert/alarm is to be escalated, or whether the alert/alarm may be disabled based on information in the therapy state data asset.

Manual therapy data may include settings (e.g., carbohydrate to insulin ratios, basal profile parameters, etc.), drug delivery parameters, recent history (e.g., timing information regarding previously delivered insulin doses), an estimate of active insulin (e.g., insulin on board), etc.

Sensor data may include data received by a medical device from one or more paired sensors (e.g., one or more glucose sensors). In some implementations, the sensor day may additionally include thresholds associated with the sensor data, such as a hypoglycemic and/or hyperglycemic threshold associated with glucose sensor data.

Therapy algorithm data may include internal data to be used by one or more algorithms implemented by the medical device. For example, an insulin pump may execute one or more algorithms to provide closed-loop control of insulin delivery. The therapy algorithm data may include internal data or internal states required by the medical device to execute the one or more algorithms.

In instances in which therapy state information is stored as a therapy state data asset, each therapy state data asset may be comprised of a set of data structures (e.g., logical structs), each having a set of fields, where the value for a given field specifies an aspect of the therapy provided to the patient at a time the therapy state data asset was created or updated. For example, a therapy state data asset may have a data structure representing glucose sensor data. Continuing with this example, the data structure representing glucose sensor data may have fields representing the most recent glucose sensor data, a hypoglycemic threshold, and/or a hyperglycemic threshold. Each of these fields may have associated values. For example, the field representing the most recent glucose sensor data may have a value corresponding to the most recent glucose sensor reading.

In some implementations, a therapy state data asset, or information indicative of a therapy state used by a medical device may be transmitted by the medical device to another device. The other device may be a controller device paired with the medical device (e.g., a mobile phone, a tablet computer, etc.), and/or a remote server or cloud device. Note that in some embodiments, the therapy state data asset or information indicative of the therapy state may be transmitted to and stored by both the controller device and a remote cloud device. The controller device and/or the cloud device may maintain latest therapy state information, which may then be provided to a replacement medical device (e.g., a second medical device). The replacement medical device may then use the received therapy state information to update therapy settings prior to provision of therapy in accordance with the updated therapy state.

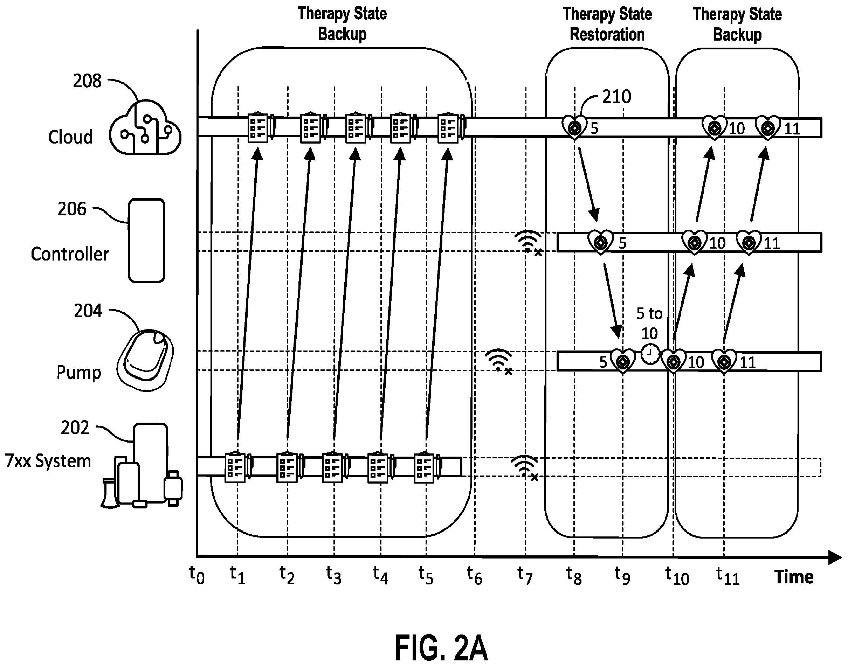

FIGS. 2A-2C provide example information flow diagrams that illustrate transfer of therapy state information across devices and restoration of a therapy state at a replacement medical device in accordance with some embodiments. Note that in each information flow diagram, the X-axis represents time.

FIG. 2A is an information flow diagram of an example of therapy state information transfer in an instance in which a first medical device 202 is a legacy medical device, or a non-compatible device, that does not utilize a therapy state data asset, and in which a second medical device 204 is a compatible device that utilizes a therapy state data asset. As illustrated, first medical device 202 delivers therapy to a patient at times t₀ - t₅. As illustrated, at each time point, as the therapy state information changes (e.g., due to updated glucose sensor readings, due to insulin being delivered by first medical device 202, due to an alert/alarm condition being triggered, etc.), the updated therapy state information is transmitted to cloud device 208. Cloud device 208 stores the received therapy state information.

Between times t₆ and t₈, first medical device 202 is replaced by second medical device 204. Note that in the example shown in FIG. 2A, second medical device 204 is a compatible device, meaning that second medical device 204 is configured to receive, parse, and utilize therapy state information packaged as a therapy state data asset. Accordingly, upon receiving information indicating that second medical device 204 will be put into service, cloud device 208 can format the therapy state information into a therapy state data asset 210. Therapy state data asset 210 is transmitted to second medical device 204 via controller device 206, as illustrated in FIG. 2A. Note that the therapy state data asset 210 is associated with time point t₅, corresponding to the last time point at which first medical device 202 transmitted therapy state information to cloud device 208. Accordingly, second medical device 204, upon receiving therapy state data asset 210 at timepoint t₉, may age, or update, particular information stored in therapy state data asset 210. In the particular example shown in FIG. 2A, second medical device 204 updates the information from times t₅ to t₁₀, corresponding to the time at which second medical device 204 begins providing therapy to the patient based on the updated therapy state information. Note that, after performing the aging or updating process, second medical device 204 also transmits the updated therapy state information to controller device 206, which transmits the updated therapy state information to cloud device 208. The transmission of updated therapy state information from second medical device 204 to cloud device 208 via controller device 206 may continue as long as second medical device 204 is utilized to provide therapy for the patient. Note that FIG. 4 describes example techniques that may be used by a second medical device (e.g., a replacement medical device) to update therapy state information based on a received therapy state data asset.

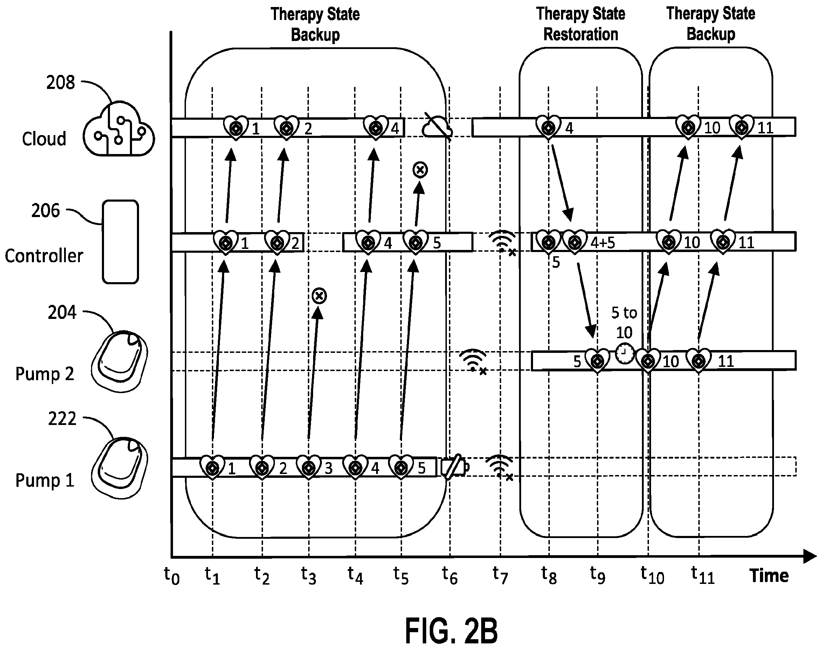

FIG. 2B illustrates an information flow diagram similar to that shown in and described above in connection with FIG. 2A. However, unlike what is shown in FIG. 2A, in FIG. 2B, first medical device 222 is a compatible device. Accordingly, rather than transmitting information indicative of a current therapy state, first medical device 222 may transmit information to cloud device 208 (via controller device 206) that is packaged as a therapy state data asset. Similar to what is shown in and described above in connection with FIG. 2A, second medical device 204 may receive a therapy state data asset responsive to second medical device 204 being placed into service, and may update the information stored in the therapy state data asset to account for the passage of time prior to utilizing the updated information to provide therapy.

Note that, in some instances, as illustrated at timepoint t₃ of FIG. 2B, a medical device may attempt to transmit a therapy state data asset to controller device 206, but the communication channel may between first medical device 222 and controller device 206 is not working. In such instances, first medical device 222 may continue attempting to transmit the therapy state data asset, and controller device 206 may receive the therapy state data asset when the communication channel is restored. A similar process occurs, e.g., at timepoint t₅, when a communication channel between controller device 206 and cloud device 208 is not functioning.

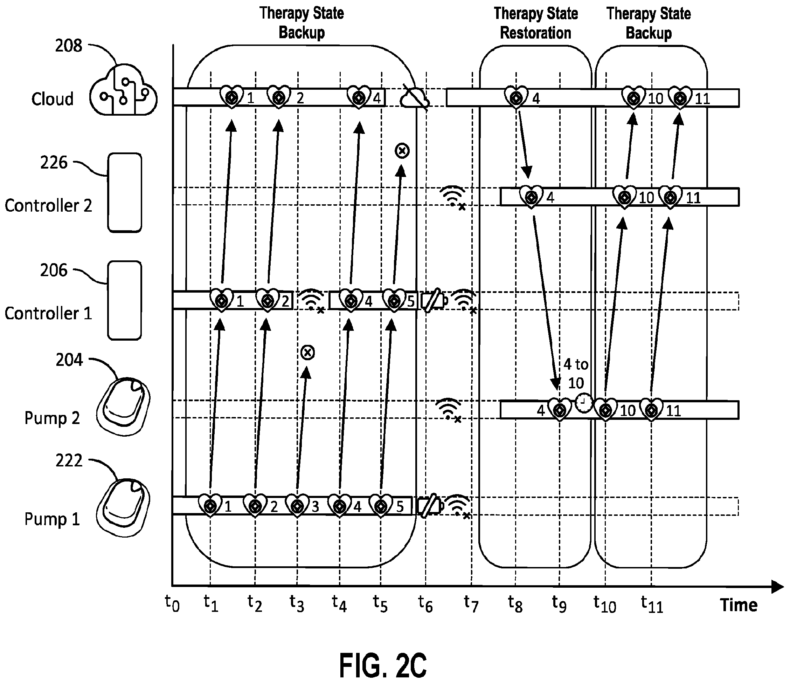

FIG. 2C illustrates an information flow diagram similar to that shown in and described above in connection with FIG. 2B. However, unlike what is shown in FIG. 2B, in the example shown in FIG. 2C, a second controller device 226, different from controller device 206 is paired with second medical device 204. Accordingly, when first medical device 222 is in operation, therapy state data assets are transmitted to cloud 208 via controller device 206. When second medical device 204 is put in service, cloud device 208 transmits the therapy state data asset to second medical device 204 via second controller device 226. The example shown in FIG. 2C illustrates the use of cloud device 208 in serving as a common backup regardless of which controller devices and/or medical devices are utilized.

As described above, in some embodiments, a controller device may serve as an intermediary to transmit therapy state information associated with therapy provided by a first medical device (which may be packaged as a therapy state data asset) to a cloud device and to receive a therapy state data asset from the cloud device and transmit the therapy state data asset to a second, replacement medical device. In some instances, therapy state information received from a medical device may be a complete therapy state data asset indicating all data fields and associated values. In other instances, the therapy state information may include differences between a previous therapy state data asset that was previously transmitted and a current therapy state provided by the medical device. Regardless of how the therapy state information is packaged and provided to the controller device, the controller device may transmit the therapy state information to a cloud device for storage and for future use (e.g., to provide to a replacement device). Note that, in instances in which a therapy state data asset is provided, e.g., to a replacement device, the therapy state data asset may include security information. For example, the security information may include a signature of the transmitting device (e.g., a signature associated with the controller device and/or the cloud device) which may be used by the recipient device (e.g., the replacement medical device) to verify a sender of the therapy state data asset. As another example, the security information may include an identifier of the recipient medical device.

FIG. 3 is a flowchart of an example process 300 for transmitting therapy state information by a controller device to a medical device in accordance with aspects of the present disclosure. An example of such a controller device is controller device 206 of FIG. 2A, controller device 226 of FIG. 2C, and/or computing device 106 of FIG. 1. In some embodiments, blocks of process 300 may be performed by one or more processors of the controller device. In some implementations, blocks of process 300 may be performed in an order other than what is shown in FIG. 3. In some embodiments, two or more blocks of process 300 may be performed substantially in parallel. In some embodiments, one or more blocks of process 300 may be omitted.

Process 300 can begin at 302 by receiving, at the controller device, an indication that a therapy state used in conjunction with a first medical device is to be transferred to a second medical device. The first medical device and the second medical device may be of the same model and/or type (e.g., as shown in and described above in connection with FIGS. 2B and 2C) and/or of different models and/or types (e.g., as shown in and described above in connection with FIG. 2A). Each medical device may be a medical device configured to provide insulin to a patient. In some embodiments, the indication that the therapy state is to be transferred may be received from the first medical device, e.g., a message indicating that the first medical device is being transitioned out of service. Alternatively, in some embodiments, the indication that the therapy state is to be transferred may be received from the second medical device, e.g., as a message indicating that the second medical device is being put into service. In some embodiments, the indication may be received from a cloud device.

At 304, process 300 can receive, at the controller device from a cloud device, information indicative of the therapy state used in conjunction with the first medical device. As shown in and described above in connection with FIGS. 2A-2C, the information indicative of the therapy state may be packaged as a therapy state data asset. For example, the information may be transmitted as a plurality of structural objects, each structural object relating to a therapy state parameter. Each structural object may include one or more fields having values representing the therapy state used in conjunction with the first medical device. As described above, by way of example, a structural object may include data relating to sensor readings obtained by a sensor communicatively coupled to the first medical device, where the fields indicate a most recent sensor reading, hyperglycemic and/or hypoglycemic thresholds, and/or a sensor identifier.

At 306, process 300 can transmit, from the controller device, the information indicative of the therapy state used in conjunction with the first medical device to the second medical device. Note that the information may be transmitted as a series of messages from the controller device to the second medical device. For example, each message may include information usable to reconstruct one or more structural objects associated with the therapy state data asset. In some embodiments, each message may be signed by the controller device (and optionally by the cloud device that provided the information to the controller device), where the signature may serve as security information that can be verified by the second medical device. Each message may include an identifier of the second medical device.

Note that, although now shown in FIG. 3, as shown in and described above in connection with FIGS. 2B and 2C, in some implementations, the controller device may have received therapy state information from the first medical device. In some embodiments, the therapy state information may have been transmitted as a therapy state data asset, e.g., as information usable to reconstruct a set of structural objects each associated with an aspect of the medical device or a therapy parameter. In some embodiments, the therapy state information transmitted by the first medical device may be a difference between a previously transmitted therapy state data asset or previously transmitted therapy state information, and a current therapy state. For example, in some embodiments, the first medical device may determine whether to transmit a complete therapy state data asset or a difference between a previously transmitted therapy state data asset and the current therapy state by determining whether the difference exceeds a different threshold. The difference threshold may be related to the amount of data that has changed, e.g., to determine how to minimize data to be transferred. In instances in which a full therapy state data asset is transmitted, the controller device may store the full therapy state data asset (and transmit the therapy state data asset to a cloud device for storage by the cloud device). In instances in which a difference is transmitted by the medical device, the controller device may store the previous therapy state data asset and the difference such that a replacement medical device may reconstruct an updated therapy state data asset based on the previous therapy state data asset and the difference.

As described above in connection with FIGS. 2A-2C, a medical device may receive, from a controller device, therapy state information associated with therapy previously provided by a previously used medical device. The therapy state information may be provided to the medical device as a therapy state data asset, as described above. The therapy state information may be received as a series of messages, each including a portion or aspect of the therapy state information. The therapy state information as received by the medical device may be associated with a first time point. The first time point may correspond to a time point at which the previously used medical device generated the therapy state information. Accordingly, the therapy state information at the time received by the medical device may represent a therapy state as provided by the previously used medical device at the first time point. The medical device may accordingly update the therapy state information, e.g., to be relevant at a current time point. Updating the therapy state information may involve updating a prediction of a current amount of insulin unmetabolized in the body of the patient (e.g., based on a duration of time that has passed between the first time point and the current time point), updating alerts or alarms that were triggered by the previously used medical device (e.g., to discard alerts and/or alarms that are no longer relevant), updating predicted levels of glucose (e.g., based on a duration of time that has passed between the first time point and the current time point), etc. The process of updating the therapy state information based on elapsed time since a time at which the therapy state information was generated by the previously used device is sometimes referred to herein as "aging."

In some implementations, prior to updating the therapy state information and/or utilizing the therapy state information, the medical device may verify and/or authenticate therapy state information received from the controller device. For example, the medical device may verify that the sender of the messages as indicated in the message signature corresponds to a known controller device and/or a known cloud device. As another example, the medical device may verify that a recipient identifier in the messages corresponds to the medical device itself. In some embodiments, responsive to determining that the therapy state information could not be verified and/or authenticated, the medical device may cause an alarm or alert to be presented. In such cases, the medical device may provide therapy using default settings or previously used settings rather than based on the received therapy state information.

FIG. 4 is a flowchart of an example process 400 for receiving therapy state information by a medical device and utilizing the received therapy state information to provide therapy in accordance with aspects of the present disclosure. An example of such a controller device is medical device 204 of FIG. 2A, 2B, and/or 2C. In some embodiments, blocks of process 400 may be performed by one or more processors of the controller device. In some implementations, blocks of process 400 may be performed in an order other than what is shown in FIG. 4. In some embodiments, two or more blocks of process 400 may be performed substantially in parallel. In some embodiments, one or more blocks of process 400 may be omitted.

Process 400 can begin at 402 by receiving, at a medical device from a controller device, therapy state information associated with therapy previously provided by a previously used medical device and associated with a first time point. Note that the medical device that receives the therapy state information may be considered a replacement medical device for the first medical device. For example, the medical device may be one that is to be placed into service, and the previously used medical device may be one that is being removed from service. The therapy state information may be received as a therapy state data asset, which may be received as a series or set of messages. In some implementations, responsive to receiving the series or set of messages, the medical device may verify the therapy state messages. For example, the medical device may verify that a signer of the messages is a known controller device (e.g., the controller device that transmitted the therapy state information) and/or a known cloud device. As another example, the medical device may verify that a recipient identifier is the medical device itself.

At 404, process 400 can update the therapy state information. For example, process 400 can update the therapy state information based on a difference between the first time point (e.g., which the therapy state information was generated by the previously used medical device) and a current time point. Updating the therapy state information may include updating a prediction of an amount of active insulin or unmetabolized insulin in the patient's body (e.g., based on timing information included in the therapy state information indicating time stamps and dosages of previously delivered insulin), discarding or updating alarm and/or alert conditions previously triggered, and/or updating a prediction of a glucose level of the patient. In some implementations, updating the therapy state information may involve obtaining one or more measurement values or parameter values corresponding to the first time point as indicated in the received therapy state information and applying the measurement values and/or parameter values to a model or algorithm to age the measurement value and/or the parameter value. By way of example, updating an amount of active insulin or unmetabolized insulin may involve obtaining insulin dosages and time points at which the insulin was delivered from the received therapy state information and providing the timing information and the dosage information to a model that generates an output indicating an amount of active insulin remaining at the current time based on a model of active insulin decay. Models that are used may be particular to the patient. For example, in some embodiments, a model may be a patient-specific physiological simulator.

At 406, process 400 can optionally establish communication with sensor device(s). For example, the medical device may use BLUETOOTH or another short-range wireless communication protocol to establish a communication channel with a glucose sensor device that provides glucose sensor readings to the medical device. Note that the communication channel may be established using information included in the received therapy state information. For example, the received therapy state information may include serial numbers of the sensor device(s), passcodes required to establish the communication channel, etc.

At 408, process 400 can provide therapy in accordance with the updated therapy state information. For example, process 400 may escalate actions based on alarms previously triggered that were not discarded as part of updating the therapy state information. As another example, process 400 may continue providing basal and/or bolus insulin dosages based on glucose sensor readings obtained from a glucose sensor paired with the medical device, e.g., as described above in connection with block 406. As yet another example, process 400 may predict glucose levels based on an updated prediction of active insulin or unmetabolized insulin, e.g., as updated in connection with block 404.

Note that, although not shown in FIG. 4, process 400 may periodically transmit updates of the therapy state used by the medical device to the controller device and/or to a cloud device. For example, the medical device may transmit the updates of the therapy state on a periodic basis (e.g., every *X* minutes). As another example, the medical device may transmit the updates of the therapy state responsive to an action occurring, e.g., responsive to delivery of insulin. As described above, updates of the therapy state may be transmitted as a therapy state data asset. In some implementations, rather than transmitting the full therapy state data asset, the medical device may determine a difference between a current therapy state and previously transmitted therapy state information (e.g., a previously transmitted therapy state data asset). Responsive to determining that the difference is below a predetermined threshold, the medical device may transmit the difference between the current therapy state and the previously transmitted therapy state information to reduce the bandwidth required.

### Example Apparatus

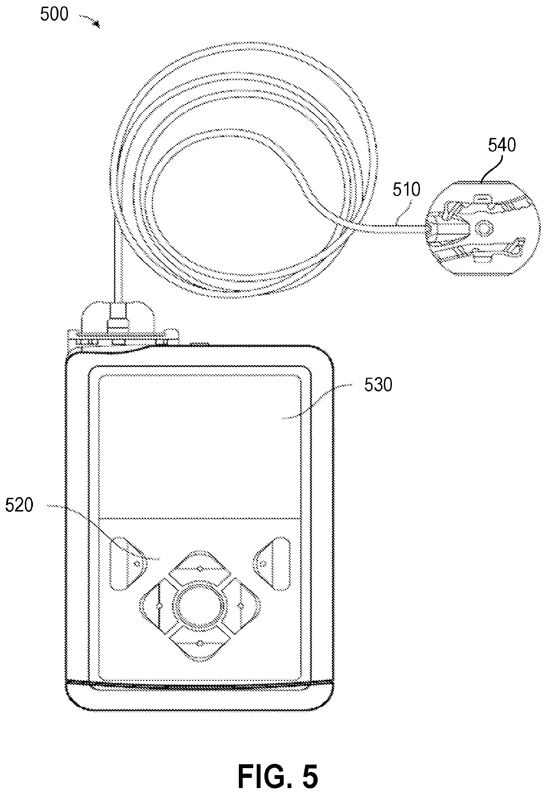

In some embodiments, therapy may be effected based on communicating a therapy determination toward a therapy delivery device. A non-limiting example of such a device is described below in connection with FIG. 5, which depicts an example insulin delivery device 500, in accordance with aspects of the present disclosure.

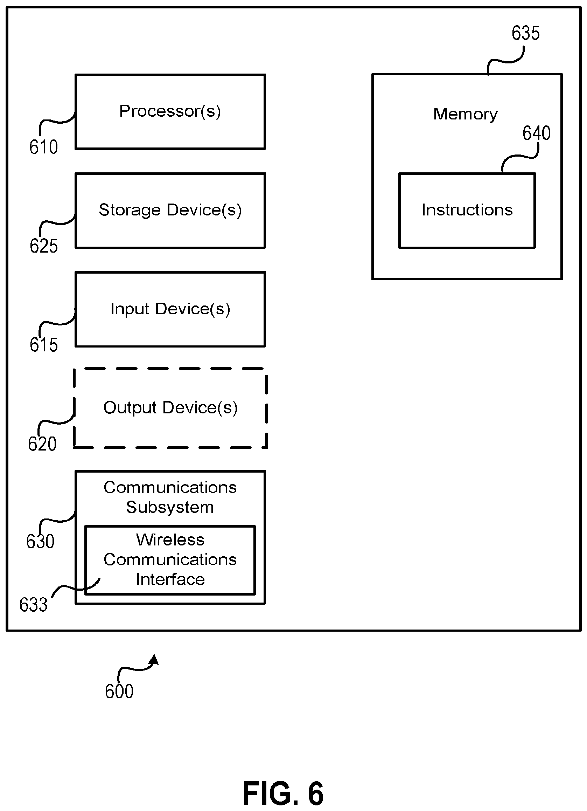

As mentioned above, therapy determinations may be communicated toward an insulin delivery device 500 (e.g., from a cloud computing system 108 via an intermediary computing device 106 communicatively coupled to the device 500). The insulin delivery device 500 may be an example of the delivery device 102 as described throughout this disclosure. In such a device, insulin delivery may be performed based on internal communication between a central computing module (e.g., a microcontroller for device 500 as a whole) and an insulin delivery module (e.g., including a motor and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., 106, FIG. 1) communicatively coupled to a remote or cloud computing system (e.g., 108, FIG. 1). The insulin delivery device 500 may communicate various event data (e.g., meal data, exercise data, and/or insulin delivery data) toward the remote or cloud computing system, which may communicate insulin delivery determinations toward the insulin delivery device 500, in some implementations. FIG. 6 further illustrates components which can be included in the insulin delivery device 500.

The insulin delivery device 500 can provide fast-acting insulin through a small tube 510 configured for fluidic connection with a cannula (not shown). The cannula may be inserted subcutaneously under a fixation dressing 540 that includes an inlet for the tube 510, an outlet for the cannula, and an adhesive surface for affixing the dressing 540 to skin. The device 500 can deliver at least two types of dosages-a basal dosage, which can be delivered periodically (e.g., every five minutes) in tiny amounts throughout the day and night, and a bolus dosage to cover an increase in blood glucose from meals and/or to otherwise correct high blood glucose levels. The depicted insulin delivery device 500 includes a user interface having button elements 520 that can be manipulated to administer a bolus of insulin, to change therapy settings, to change user preferences, to select display features, and the like. The insulin delivery device 500 also includes a display device 530 that can be used to present various types of information or data to the user (such as a notification or an alert of the type described above). In accordance with aspects of the present disclosure, a user of the insulin delivery device 500 may use the button elements 520 to input certain event data (e.g., event type, event start time, event details, etc.), and the user inputs can be confirmed using the display device 530. The depicted insulin delivery device 500 of FIG. 5 is merely provided by way of example, and other types of insulin delivery devices and other techniques different from those described above are contemplated to be within the scope of the present disclosure.

FIG. 6 is a block diagram of an embodiment of a computer system 600, which can be utilized in embodiments as described herein. It should be noted that FIG. 6 is meant only to provide a generalized illustration of various components, any or all of which may be utilized as appropriate. In addition, it can be noted that components illustrated by FIG. 6 can be localized to a single device (e.g., delivery device 102, monitoring device 104, computing device 106, or computing system 108) and/or distributed among various networked devices, which may be disposed at different geographical locations.

In some embodiments, the computer system 600 may include hardware elements that can be electrically coupled via a bus (or may otherwise be in communication, as appropriate). The hardware elements may include processor(s) 610, which may comprise, without limitation, one or more microcontroller(s), one or more microprocessor(s), one or more general-purpose processors, one or more special-purpose processors (such as digital signal processing chips, graphics acceleration processors, and/or the like), and/or other processing structure, which can be configured to perform one or more of the methods or functionalities described herein.

In some embodiments, the computer system 600 also may include one or more input devices 615, which may comprise, without limitation, button elements 520, a microphone, a glucose sensor, and/or the like. The computer system 600 may also include one or more output devices 620, which may comprise without limitation a display device (e.g., 530), a speaker, a buzzer, and/or the like.

In some embodiments, the computer system 600 may further include one or more non-transitory storage devices 625, which can include, without limitation, local and/or network accessible storage, and/or may comprise, without limitation, a disk drive, a drive array, an optical storage device, a solid-state storage device, such as a read-access memory (RAM) and/or read-only memory (ROM), which can be programmable, flash-updateable, and/or the like. Such storage devices may be configured to implement any appropriate data stores, including without limitation, various file systems, database structures, and/or the like. Such data stores may include database(s) and/or other data structures used to store and administer messages and/or other information to be sent to one or more other components or external devices.

In some embodiments, the computer system 600 may also include a communications subsystem 630, which may implement wireless communication technologies managed and controlled by a wireless communication interface 633. Additionally or alternatively, communications subsystem 630 may implement wired technologies (such as Ethernet, coaxial communications, universal serial bus (USB), or the like). The wireless communication interface 633 may comprise one or more wireless transceivers that may send and receive wireless signals (e.g., signals according to Bluetooth, Bluetooth Low Energy (BLE)). Thus, the communications subsystem 630 may comprise a modem, a network card (wireless or wired), an infrared communication device, a wireless communication device, and/or a chipset, and/or the like, which may enable the computer system 600 to communicate with any device discussed with respect to FIG. 1, including delivery device 102, monitoring device 104, computing device 106, and/or a cloud computing system 108 as described herein. Hence, the communications subsystem 630 may be used to receive and send data (e.g., SG, Ip, insulin delivery information) as described in the embodiments herein.

In some embodiments, the computer system 600 will further comprise a working memory 635, which may comprise a RAM or ROM device, as described above. Software elements, shown as being located within the working memory 635, may comprise computer-readable and computer-executable instructions 640; device drivers; executable libraries; and/or other code, which may comprise computer programs used in various embodiments and/or may be designed to implement methods and/or configure systems in accordance with embodiments described herein. Merely by way of example, one or more operations described with respect to the methods or functionalities discussed above might be implemented as code and/or instructions executable by a computer (and/or a processor within a computer). Such code and/or instructions can be used to configure and/or adapt a general-purpose computer (or other device) to perform one or more operations in accordance with the described methods.

In some embodiments, a set of these instructions and/or code may be stored on a non-transitory computer-readable storage medium, such as the storage device(s) 625 described above. In some cases, the storage medium might be incorporated within a computer system, such as computer system 600. In other embodiments, the storage medium might be separate from a computer system (e.g., a removable medium, such as an optical disc) and/or provided in a downloadable installation package, such that the storage medium can be used to program, configure, and/or adapt a general purpose computer with the instructions and/or code stored thereon. These instructions might take the form of executable code, which is executable by the computer system 600, and/or might take the form of source and/or installable code, which, upon compilation and/or installation on the computer system 600 (e.g., using any of a variety of generally available compilers, installation programs, compression/decompression utilities, etc.), then takes the form of executable code.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

In view of this description embodiments may include different combinations of features. Implementation examples are described in the following numbered clauses:
Example 1: A method comprising: receiving, at a controller device, an indication that a therapy state used in conjunction with a first medical device is to be transferred to a second medical device; receiving, at the controller device from a cloud device, information indicative of the therapy state used in conjunction with the first medical device; and transmitting, from the controller device, the information indicative of the therapy state used in conjunction with the first medical device to the second medical device.
Example 2: The method of example 1, wherein transmitting the information indicative of the therapy state comprises transmitting a plurality of messages from the controller device to the second medical device, wherein each message of the plurality of messages includes security information.
Example 3: The method of example 2, wherein the security information includes an identifier of the controller device and an identifier of the second medical device.
Example 4: The method of any one of examples 1-3, wherein the information indicative of the therapy state used in conjunction with the first medical device comprises: connectivity data indicating communication information to communicatively couple the second medical device to one or more other devices; timestamp information indicative of a time the therapy state used in conjunction with the first medical device was stored; alarm or notification data; drug delivery parameters; sensor data; therapy delivery algorithm data; or any combination thereof.
Example 5: The method of any one of examples 1-4, wherein the information indicative of the therapy state is transmitted as a plurality of structural objects, each structural object relating to a therapy state parameter and each structural object comprising one or more fields having values representing the therapy state used in conjunction with the first medical device.
Example 6: The method of any one of examples 1-5, further comprising, prior to receiving the indication that the therapy state used in conjunction with the first medical device is to be transferred to the second medical device: receiving the information indicative of the therapy state used in conjunction with the first medical device; and transmitting the received information indicative of the therapy state to the cloud device for storage by the cloud device.
Example 7: The method of any one of examples 1-6, wherein the first medical device and the second medical device are the same type and model of medical device.
Example 8: The method of any one of examples 1-7, wherein the first medical device and the second medical device are different types and/or models of medical devices.
Example 9: A method comprising: receiving, at a medical device, therapy state information associated with therapy provided by a previously used medical device, wherein the therapy state information is associated with a first time point at which the therapy was provided by the previously used medical device; updating the therapy state information by the medical device; and providing therapy, by the medical device, in accordance with the updated therapy state information.
Example 10: The method of example 9, wherein updating the therapy state information by the medical device is based at least in part on a difference between a second time point at which the medical device received the therapy state information and the first time point.
Example 11: The method of example 10, wherein updating the therapy state information comprises updating one or more alarm conditions indicated in the therapy state information based on the difference between the second time point and the first time point.
Example 12: The method of any one of examples 10 or 11, wherein updating the therapy state information comprises updating an estimate of an amount of insulin in a patient associated with the medical device based on the difference between the second time point and the first time point.
Example 13: The method of any one of examples 9-12, further comprising, prior to updating the therapy state information, verifying a security of the therapy state information received.
Example 14: The method of example 13, wherein verifying the security of the therapy state information received comprises verifying an authenticity of a sender of the therapy state information.
Example 15: The method of any one of examples 9-14, wherein the therapy state information is received from a controller device communicatively coupled to the medical device.
Example 16: The method of example 15, further comprising transmitting, to the controller device, information indicative of the updated therapy state information.
Example 17: The method of example 16, wherein transmitting the information indicative of the updated therapy state information comprises transmitting a difference between a previous therapy state and the updated therapy state information.
Example 18: The method of example 17, further comprising determining that the updated therapy state information is to be transmitted using a difference between the previous therapy state and the updated therapy state information by comparing the difference to a threshold difference and determining the difference is less than the threshold difference.
Example 19: The method of any one of examples 9-18, further comprising using connectivity information included in the therapy state information to establish a communication channel with at least one sensor device.
Example 20: A system comprising: one or more processors; and one or more processor-readable media storing instructions which, when executed by one or more processors. The one or more processors cause performance of: receiving, at a controller device, an indication that a therapy state used in conjunction with a first medical device is to be transferred to a second medical device; receiving, at the controller device from a cloud device, information indicative of the therapy state used in conjunction with the first medical device; and transmitting, from the controller device, the information indicative of the therapy state used in conjunction with the first medical device to the second medical device.

Further disclosed herein is the subject-matter of the following clauses:
1. A method comprising:
   receiving, at a controller device, an indication that a therapy state used in conjunction with a first medical device is to be transferred to a second medical device;
   receiving, at the controller device from a cloud device, information indicative of the therapy state used in conjunction with the first medical device; and
   transmitting, from the controller device, the information indicative of the therapy state used in conjunction with the first medical device to the second medical device.
2. The method of clause 1, wherein transmitting the information indicative of the therapy state comprises transmitting a plurality of messages from the controller device to the second medical device, wherein each message of the plurality of messages includes security information.
3. The method of clause 1 or 2, wherein the security information includes an identifier of the controller device and an identifier of the second medical device.
4. The method of clause 1 or of any of clauses 1 to 3, wherein the information indicative of the therapy state used in conjunction with the first medical device comprises: connectivity data indicating communication information to communicatively couple the second medical device to one or more other devices; timestamp information indicative of a time the therapy state used in conjunction with the first medical device was stored; alarm or notification data; drug delivery parameters; sensor data; therapy delivery algorithm data; or any combination thereof.
5. The method of clause 1 or of any of clauses 1 to 4, wherein the information indicative of the therapy state is transmitted as a plurality of structural objects, each structural object relating to a therapy state parameter and each structural object comprising one or more fields having values representing the therapy state used in conjunction with the first medical device.
6. The method of clause 1 or of any of clauses 1 to 5, further comprising, prior to receiving the indication that the therapy state used in conjunction with the first medical device is to be transferred to the second medical device:
   receiving the information indicative of the therapy state used in conjunction with the first medical device; and
   transmitting the received information indicative of the therapy state to the cloud device for storage by the cloud device.
7. The method of clause 1 or of any of clauses 1 to 6, wherein the first medical device and the second medical device are the same type and model of medical device.
8. The method of clause 1 or of any of clauses 1 to 7, wherein the first medical device and the second medical device are different types and/or models of medical devices.
9. A method comprising:
   receiving, at a medical device, therapy state information associated with therapy provided by a previously used medical device, wherein the therapy state information is associated with a first time point at which the therapy was provided by the previously used medical device;
   updating the therapy state information by the medical device; and
   providing therapy, by the medical device, in accordance with the updated therapy state information.
10. The method of clause 9, wherein updating the therapy state information by the medical device is based at least in part on a difference between a second time point at which the medical device received the therapy state information and the first time point.
11. The method of clause 9 or 10, wherein updating the therapy state information comprises updating one or more alarm conditions indicated in the therapy state information based on the difference between the second time point and the first time point.
12. The method of clause 10 or of any of clauses 9 to 11, wherein updating the therapy state information comprises updating an estimate of an amount of insulin in a patient associated with the medical device based on the difference between the second time point and the first time point.
13. The method of clause 9 or of any of clauses 9 to 12, further comprising, prior to updating the therapy state information, verifying a security of the therapy state information received.
14. The method of clause 13 or of any of clauses 9 to 13, wherein verifying the security of the therapy state information received comprises verifying an authenticity of a sender of the therapy state information.
15. The method of clause 9 or of any of clauses 9 to 14, wherein the therapy state information is received from a controller device communicatively coupled to the medical device.
16. The method of clause 15 or of any of clauses 9 to 15, further comprising transmitting, to the controller device, information indicative of the updated therapy state information.
17. The method of clause 16 or of any of clauses 9 to 16, wherein transmitting the information indicative of the updated therapy state information comprises transmitting a difference between a previous therapy state and the updated therapy state information.
18. The method of clause 17 or of any of clauses 9 to 17, further comprising determining that the updated therapy state information is to be transmitted using a difference between the previous therapy state and the updated therapy state information by comparing the difference to a threshold difference and determining the difference is less than the threshold difference.
19. The method of clause 9 or of any of clauses 9 to 18, further comprising using connectivity information included in the therapy state information to establish a communication channel with at least one sensor device.
20. A system comprising:
   one or more processors; and
   one or more processor-readable media storing instructions which, when executed by one or more processors, cause performance of:
      receiving, at a controller device, an indication that a therapy state used in conjunction with a first medical device is to be transferred to a second medical device;
      receiving, at the controller device from a cloud device, information indicative of the therapy state used in conjunction with the first medical device; and
      transmitting, from the controller device, the information indicative of the therapy state used in conjunction with the first medical device to the second medical device.

## Claims

1. A method comprising:
receiving, at a controller device, an indication that a therapy state used in conjunction with a first medical device is to be transferred to a second medical device;
receiving, at the controller device from a cloud device, information indicative of the therapy state used in conjunction with the first medical device; and
transmitting, from the controller device, the information indicative of the therapy state used in conjunction with the first medical device to the second medical device.

2. The method of claim 1, wherein transmitting the information indicative of the therapy state comprises transmitting a plurality of messages from the controller device to the second medical device, wherein each message of the plurality of messages includes security information,
particularly
wherein the security information includes an identifier of the controller device and an identifier of the second medical device.

3. The method of claim 1 or 2, wherein the information indicative of the therapy state used in conjunction with the first medical device comprises: connectivity data indicating communication information to communicatively couple the second medical device to one or more other devices; timestamp information indicative of a time the therapy state used in conjunction with the first medical device was stored; alarm or notification data; drug delivery parameters; sensor data; therapy delivery algorithm data; or any combination thereof.

4. The method of one of claims 1 to 3, wherein the information indicative of the therapy state is transmitted as a plurality of structural objects, each structural object relating to a therapy state parameter and each structural object comprising one or more fields having values representing the therapy state used in conjunction with the first medical device.

5. The method of one of claims 1 to 4, further comprising, prior to receiving the indication that the therapy state used in conjunction with the first medical device is to be transferred to the second medical device:
receiving the information indicative of the therapy state used in conjunction with the first medical device; and
transmitting the received information indicative of the therapy state to the cloud device for storage by the cloud device.

6. The method of one of claims 1 to 5, wherein the first medical device and the second medical device are the same type and model of medical device,
or
wherein the first medical device and the second medical device are different types and/or models of medical devices.

7. A method comprising:
receiving, at a medical device, therapy state information associated with therapy provided by a previously used medical device, wherein the therapy state information is associated with a first time point at which the therapy was provided by the previously used medical device;
updating the therapy state information by the medical device; and
providing therapy, by the medical device, in accordance with the updated therapy state information.

8. The method of claim 7, wherein updating the therapy state information by the medical device is based at least in part on a difference between a second time point at which the medical device received the therapy state information and the first time point.

9. The method of claim 8, wherein updating the therapy state information comprises updating one or more alarm conditions indicated in the therapy state information based on the difference between the second time point and the first time point.

10. The method of claim 8 or 9, wherein updating the therapy state information comprises updating an estimate of an amount of insulin in a patient associated with the medical device based on the difference between the second time point and the first time point.

11. The method of one of claims 7 to 10, further comprising, prior to updating the therapy state information, verifying a security of the therapy state information received, particularly
wherein verifying the security of the therapy state information received comprises verifying an authenticity of a sender of the therapy state information.

12. The method of one of claims 7 to 11, wherein the therapy state information is received from a controller device communicatively coupled to the medical device,
particularly
further comprising transmitting, to the controller device, information indicative of the updated therapy state information.

13. The method of claim 12, wherein transmitting the information indicative of the updated therapy state information comprises transmitting a difference between a previous therapy state and the updated therapy state information,
particularly
further comprising determining that the updated therapy state information is to be transmitted using a difference between the previous therapy state and the updated therapy state information by comparing the difference to a threshold difference and determining the difference is less than the threshold difference.

14. The method of one of claims 7 to 13, further comprising using connectivity information included in the therapy state information to establish a communication channel with at least one sensor device.

15. A system comprising:
one or more processors; and
one or more processor-readable media storing instructions which, when executed by one or more processors, cause performance of:
receiving, at a controller device, an indication that a therapy state used in conjunction with a first medical device is to be transferred to a second medical device;
receiving, at the controller device from a cloud device, information indicative of the therapy state used in conjunction with the first medical device; and
transmitting, from the controller device, the information indicative of the therapy state used in conjunction with the first medical device to the second medical device.
